# EUROPEAN PATENT SPECIFICATION

(11) **EP 4 575 001 B1**
(45) Date of publication and mention of the grant of the patent: **18.11.2026**
(21) Application number: 23398028.3
(22) Date of filing: 19.12.2023
(51) Int. Cl.: C12Q 1/6888

(54) **A METHOD OF GENOTYPING SINGLE NUCLEOTIDE POLYMORPHISMS THAT ARE ASSOCIATED WITH THE PRODUCTION AND QUALITY OF SHEEP MILK**
VERFAHREN ZUR GENOTYPISIERUNG VON EINZELNUKLEOTIDPOLYMORPHISMEN, DIE MIT DER PRODUKTION UND QUALITÄT VON SCHAFSMILCH ASSOZIIERT SIND
PROCÉDÉ DE GÉNOTYPAGE DE POLYMORPHISMES MONONUCLÉOTIDIQUES ASSOCIÉS À LA PRODUCTION ET À LA QUALITÉ DU LAIT DE MOUTON

(43) Date of publication of application: 25.06.2025
(73) Proprietor: Instituto Nacional de Investigação Agrária e Veterinária I.P., 2780-157 Oeiras (PT)
(72) Inventor: Belo, Ana Teresa Carmona, 2005-424 Vale de Santarém (PT); Fernandes Marques, Maria do Rosário, 2005-424 Vale de Santarém (PT)
(74) Representative: Couto, Cláudia

(56) References cited:
- DO ROSARIO MARQUES MARIA ET AL: "Associations of SNPs in the ovine prolactin and prolactin receptor genes with milk traits in Assaf dairy sheep Project ALT20-03-0145-FEDER-000019", 10 July 2019 (2019-07-10), XP093174447, Retrieved from the Internet <URL:https://www.researchgate.net/publication/334376413> DOI: 10.13140/RG.2.2.31948.16004
- ROSÁRIO MARQUES MARIA ET AL: "Effects of nonsynonymous SNPs at GH2-N and GHR genes on coagulation properties of Assaf ewes' milk", 70TH ANNUAL MEETING OF THE EUROPEAN FEDERATION OF ANIMAL SCIENCE, GHENT , BELGIUM, 30 August 2019 (2019-08-30), XP093174593, Retrieved from the Internet <URL:https://docs.eaap.org/2019/theater/15/1530_Marques.pdf>
- MARQUES M ET AL: "EFEITOS DOS POLIMORFISMOS GENÉTICOS NA PRODUÇÃO E QUALIDADE DO LEITE DE OVELHA", VIDA RURAL, 1 September 2021 (2021-09-01), pages 68 - 74, XP093174634, Retrieved from the Internet <URL:https://www.iniav.pt/images/publicacoes/2021/Efeitos_dos_polimorfismos_geneticos.pdf>
- DETTORI MARIA L ET AL: "Association between theGHR,GHRHR, andIGF1gene polymorphisms and milk yield and quality traits in Sarda sheep", JOURNAL OF DAIRY SCIENCE, AMERICAN DAIRY SCIENCE ASSOCIATION, US, vol. 101, no. 11, 23 August 2018 (2018-08-23), pages 9978 - 9986, XP085510017, ISSN: 0022-0302, DOI: 10.3168/JDS.2018-14914

## Description

### Technical field

This application relates to a method of genotyping Single Nucleotide Polymorphisms (SNPs) associated with the production and quality of sheep milk.

### Background art

Recent studies based on large portions of the genome have revealed the existence of loci associated with quantitative traits such as lactation persistence in dairy cows (Pryce et al., 2010) and dairy sheep (Jonas et al., 2011). Milk production, the persistence of different traits related to milk production and quality, and the length of lactation are traits likely to be controlled by different genes on different chromosomes (Gutiérrez-Gil et al., 2009; Mateescu and Thonney, 2010; Jonas et al., 2011).

Milk production, as well as lactation persistence and the production of the various milk constituents are controlled by several genes of the somatotropic axis, including the growth hormone gene - GH, previously associated with milk production and quality in Serra da Estrela (Marques et al., 2006) and Sarda sheep breeds (Vacca et al., 2013), some of its transcription factors, such as STAT3, STAT5A and STAT5B, as well as the insulin-like growth factor binding proteins IGF2BP1 and IGFBP4, suppressor of cytokine signaling 3 (SOCS3), tachykinin 4 (TAC4) and a hemokine important in modulating prolactin expression during lactation (Pisera et al., 1998), also involved in glucose metabolism and anti-apoptotic signaling mechanisms and cell survival, among others. Molecular markers have been found in the GH gene itself, prolactin, gene receptors and transcription factors of the main pathways known to be regulated by GH, i.e., the GHR-JAK2-STAT-IGF-1 axis (Carter-Su et al., 2000), the IGF1-IGFR-MAPK signaling cascade (Fleming et al., 2005) and the IGF1-IGFR-PI3K-mTOR axis (Sciascia et al., 2013). All significantly affect the normal development of the mammary gland (Bauman, 1999; Falaki et al., 1997; Lucy et al., 1993; Kann et al., 1999), milk production (Dettori et al., 2013; Marques et al., 2006; Vacca et al., 2013) and have been implicated in the control of transcription factors involved in regulating the expression of casein and whey protein genes (Rosen et al., 1999). Animal selection based on such markers offers enormous potential for improving sheep milk production. In fact, GH is also known to indirectly influence the synthesis and secretion of milk components, either by increasing blood flow and nutrient availability to the mammary gland (Bauman, 1999; Etherton and Bauman, 1998), or through the stimulation of IGF-1 secretion by the liver and mammary stromal cells (Akers et al., 2005; Capuco et al., 2001). These physiological stimuli increase the proliferation of epithelial cells or the secretory activity of the mammary gland during lactation (Boutinaud et al., 2003; Capuco et al., 2001), indicating the involvement of signaling pathways that regulate the number of cells and their rate of renewal (Fleming et al., 2005; Modha et al., 2004; Sakamoto et al., 2005; Sciascia et al., 2013), RNA transcription and post-transcriptional miRNAs (Li et al., 2012), and protein synthesis (Burgos and Cant, 2010; Clarkson et al., 2006). After peak lactation, the fall in milk production (concomitant with the decrease of GH in plasma) and the reduction in lactation persistence appear to be related to the increase in insulin concentration and greater responsiveness of adipose tissue to insulin. This means that any increase in blood glucose stimulates the action of insulin, favoring the use of glucose in the anabolic processes of peripheral tissues, but not by the mammary gland, which affects milk production and can lead to differences in milk composition, and therefore in its suitability for coagulation and cheese yield (Martins et al., 2009).

The SNaPshot^{®} Multiplex System is an allele-specific primer extension-based technology developed for the analysis of SNPs. Following a first target amplification from genomic DNA, followed by purification of the amplified DNA, numerous SNPs can be genotyped simultaneously in a single reaction due to its multiplexing capabilities. The SNaPshot primer targets a sequence immediately upstream of the SNP site and is expanded by a single base in the presence of all four fluorescently labeled dideoxy nucleotides (ddNTPs). Each fluorescent ddNTP emits a particular wavelength, which is converted into a specific colour for each base. The product is the same size as the initial probe plus one fluorescent base. The reactions are run on a genetic analyser, and genotypes are determined by the colour and location of the emitted fluorescence peak. The ability to use unlabelled, user-defined primers allows to incorporate SNPs of interest cost-effectively. The Multiplex Ready Reaction Mix (included in the system) helps ensure robust, reproducible analyses of multiplexed samples, saving time and money. Since it requires little optimization, primer design and DNA template purification can significantly affect genotyping accuracy.

Document "Associations of SNPs in the ovine prolactin and prolactin receptor genes with milk traits in Assaf dairy sheep", Do Rosario et al., discloses prolactin gene and prolactin receptor gene polymorphisms, a multiplex of 9 SNPs, however, it doesn't mention the primers or the improved method steps of this patent application.

Document "Effects of nonsynonymous SNPs at GH2-N and GHR genes on coagulation properties of Assaf ewes' milk", Rosário, M. et al., discloses 6 SNP multiplex which are used for the analysis of milk production and quality, however, it doesn't mention the primers or the improved method steps of this patent application.

Document "Efeitos dos polimorfismos genéticos na produção e qualidade do leite de ovelha", Marques, M. et al., discloses a group of 14 SNPs for the analysis of milk production and quality, however, it doesn't mention the primers or the improved method steps of this patent application.

### Summary

The present invention relates to a method of genotyping single nucleotide polymorphisms in somatotropic axis genes that are associated with the production and quality of sheep milk comprising the following steps:
- Extracting genomic DNA from a biological sample obtained from sheep;
- Amplifying the aforementioned DNA by multiplex PCR comprising:
   - A first multiplex PCR comprising the amplification of the copy GH2-N of the growth hormone, exon 10 of the growth hormone receptor and exon 8 of the insulin-like growth factor 1 receptor gene fragments using the primers comprising SEQ ID No. 1 to 6;

   - A second multiplex PCR comprising the amplification of the 5'-UTR region, exon 1, 2 and 3 of prolactin, and the exon 10 of the prolactin receptor gene fragments using the primers comprising SEQ ID No. 7 to 16;
   - A third multiplex PCR comprising the amplification of the 5'-UTR region of the insulin-like growth factor 1 and the exon 3 of the insulin-like growth factor 1 receptor gene fragments using the primers comprising SEQ ID No. 17 to 20;
- Preparing a sample comprising the products of the three multiplex PCR;
- Enzymatically purifying the sample with exonuclease I and shrimp alkaline phosphatase;
- Performing an extension reaction on the sample using the primers comprising SEQ ID No. 21 to 36;
- Adding shrimp alkaline phosphatase, purifying the extension reaction product and performing an enzymatic inactivation;
- Analysing an interpreting the results to obtain the genotypes of the SNPs.

In one embodiment, the biological sample is collected after the sheep's birth or throughout the live of the sheep.

In one embodiment, the biological sample is selected from blood, milk, semen, or tissue collected from a sheep.

In one embodiment, the DNA sample comprises at least one gene from the group of GH (NCBI Gene ID 443329), GHR (NCBI Gene ID 443333), PRL (NCBI Gene ID 443317), PRLR (NCBI Gene ID 554331), IGF1 (NCBI Gene ID 443318) or IGF1-R (NCBI Gene ID 443515).

In one embodiment, the single nucleotide polymorphisms is at least one selected from the list comprising: g.597T>C, g.717C>T, g.1024T>C in the GH gene; rs1086611503, rs595567866, rs597181420 in the GH receptor gene; rs400524445, rs1094731965 in the IGF1 receptor gene; g.23731G>T, rs412263261, rs408430940, rs406266481 in the Prolactin gene; rs604784916, rs600947105 in the PRL receptor gene; rs401028781, rs412597723 in the IGF1 gene.

In one embodiment, the first multiplex PCR is carried out with 1U of Taq DNA polymerase, 1 µM of each primer and 10 ng/µL of DNA sample, the enzyme activation is carried out for 2 minutes at 94°C, followed by 30 cycles of DNA denaturation for 10 seconds at 94°C, primer annealing for 20 seconds at 63.2°C, an extension for 1 minute at 72°C, and a final extension for 10 minutes at 72°C.

In one embodiment, the second multiplex PCR is carried out with 0.625 U of DFS-Taq DNA Polymerase, 1 µM of each primer and 5 ng/µL of DNA sample, the enzyme activation is carried out for 2 minutes at 94°C, followed by 30 cycles of DNA denaturation for 10 seconds at 94°C, primer annealing for 20 seconds at 63°C, an extension for 1 minute at 72°C, and a final extension for 10 minutes at 72°C.

In one embodiment, the third multiplex PCR is carried out with 1 U of DNA Polymerase, 0.6 µM of each primer, 2 mM of MgCl₂ and 10 ng/µL of DNA sample, the enzyme activation is carried out for 15 minutes at 95°C, followed by 30 cycles of DNA denaturation for 30 seconds at 95°C, primer annealing for 2 minutes at 65.9°C, an extension for 2 minute at 72°C, and a final extension for 10 minutes at 72°C.

In one embodiment, the products of the three multiplex PCR are combined in a ratio of 1:1.

In one embodiment, the first purification step of the sample is carried out for 15 minutes at 37°C, followed by 15 minutes at 80°C for enzymatic inactivation.

In one embodiment, the extension reaction is carried out with 25 cycles of 90°C for 10 seconds, 50°C for 5 seconds, 60°C for 3 seconds and a final extension step at 72°C for 2 minutes.

In one embodiment, the purification of the extension reaction product is carried out for 1 hour at 37°C, followed by 15 minutes at 75°C for enzymatic inactivation.

In one embodiment, the resulting products are analyzed by capillary electrophoresis.

The invention also relates to a kit for genotyping single nucleotide polymorphisms in somatotropic axis genes that are associated with the production and quality of sheep milk, wherein the kit comprises the primer set SEQ ID No. 1 to 36 for use in the method.

### General description

The present application related to the field of recombinant DNA technology and discloses a methodology suitable for identifying 16 genetic markers, which are single nucleotide polymorphisms (SNPs), distributed across six somatotropic axis genes: GH, GHR, PRL, PRLR, IGF1 and IGF1-R, associated with sheep milk production and quality.

This invention is useful for early identification and selection of breeding animals with high genetic merit for producing milk with high quality.

The present method relies on the SNaPshot method for the detection of 16 SNPs distributed among the genes for growth hormone (GH) and its receptor (GHR), prolactin (PRL) and its receptor (PRLR), insulin-like growth factor 1 (IGF1) and its receptor (IGF1-R). The characterization of the SNPs to be detected within the scope of this invention is shown in Table 1A and 1B.

**Table 1A. Characterization of the SNPs to genotype.**

| **SNP No.** | **Gene** | **Name** | **Gene ID from NCBI***** | **Chr** | **Location** | **SNP** |
|---|---|---|---|---|---|---|
| **1** | *GH* | Growth hormone | 443329 | 11 | NC_040262.1 (14849149..14850884) * | g.597T>C |
| **2** | | | | | | g.717C>T |
| **3** | | | | | | g.1024T>C |
| **4** | *GHR* | GH receptor | 443333 | 16 | NC_040267.1 (33625221..33923727, complement) * | rs1086611503 |
| **5** | | | | | | rs595567866 |
| **6** | | | | | | rs597181420 |
| **16** | *IGF1-R* | IGF1 receptor | 443515 | 18 | NC_040269.1 (7575639..7879425)* | rs400524445 |
| **7** | | | | | | rs1094731965 |
| **8** | *PRL* | Prolactin | 443317 | 20 | NC_040271.1 (38543004..38551470, complement) * | g.23731G>T |
| **9** | | | | | | rs412263261 |
| **10** | | | | | | rs408430940 |
| **11** | | | | | | rs406266481 |
| **12** | *PRLR* | PRL receptor | 554331 | 16 | 16: 41118082-41325423 ** | rs604784916 |
| **13** | | | | | | rs600947105 |
| **14** | *IGF1* | Insulin-like growth factor 1 | 443318 | 3 | NC_040254.1 (183945231..184028095, complement) * | rs401028781 |
| **15** | | | | | | rs412597723 |

| | | | | | | |
|---|---|---|---|---|---|---|
| * Oar_rambouillet_v1.0(GCF_002742125.1) ** Oar_rambouillet_v1.0:CM008487.1 *** National Center for Biotechnology Information - last accessed in November 2023. | | | | | | |

**Table 1B. Continuation of Table 1A - Characterization of the SNPs to genotype.**

| **SNP No.** | **Location** | **Sequence** | **SNP position** | **Alele** | **aminoacid** |
|---|---|---|---|---|---|
| **1** | Exon 2 | X12546 | 597 | T>C | Leu11Pro |
| **2** | | | 717 | C>T | Ala51Val |
| **3** | Exon 3 | | 1024 | T>C | Phe78Leu |
| **4** | Exon 10 | NC_019473.1 | 31835095 | C>T | Ser380Pro |
| **5** | | | 31835059 | C>T | Glu392Lys |
| **6** | | | 31834648 | C>T | Ala529Thr |
| **16** | Exon 3 | NC_019475.1 | 7162694 | G>A | Gly74Arg |
| **7** | Exon 8 | | 7178705 | G>A | Met534Arg |
| **8** | 5'UTR | AMGL02030943.1 | 23731 | G>T | - |
| **9** | Exon 1 | NC_019477.1 | 34266402 | A>C | Val7Gly |
| **10** | Exon 2 | | 34263597 | A>G | Thr33= |
| **11** | Exon 3 | | 34261223 | T>G | Arg75= |
| **12** | Exon 10 | NC_019473.1 | 39027431 | C>T | Arg351Trp |
| **13** | | | 39027963 | C>T | Pro528Leu |
| **14** | 5'-UTR | NC_019460.1 | 171328400 | C>G | - |
| **15** | | | 171328575 | G>A | - |

Based on the identification of these 16 SNP in DNA samples from biological material obtained of sheep, it is possible to define their genotypes for each SNP and their haplotypes, which have been significantly associated with milk traits: milk yield, fat protein, lactose and total solids contents; and milk coagulation properties. Thus, depending on the breeders' selection objectives, they can use these genotyping results to select, as soon as possible, the most suitable ewes and/or ram to breed.

In the context of the present invention, biological material is understood as a material derived from, or produced by, biological organisms, any material containing genetic information.

Examples of statistical analysis that revealed associations between phenotypic characteristics (milk production and quality, milk clotting properties, etc) and the genotyped SNPs as follows:
- SNP X12546:g.597T>C genotypes affected gel firmness after a 2R period (AR; P<0.01) (Marques et al., 2019c);
- SNP X12546:g.717C>T genotypes affected significantly milk yield (P<0.05) (Marques et al., 2006);
- SNP X12546:g.1024T>C genotypes affected AR (P<0.05) (Marques et al., 2019c);
- rs1086611503 genotypes affected milk protein (TP; P<0.05) (Marques et al., 2019c);
- SNP rs597181420 genotypes affected pH (P<0.01) and clotting time (R; P<0.01) (Marques et al., 2019c);
- SNP rs1086611503 / rs595567866 / rs597181420 haplotypes affected R (P<0.05) and AR (P<0,01) (Marques et al., 2019c);
- SNP AMGL02030943.1:g.23731G>T genotypes affected milk production throughout lactation (P<0.01), and R (P<0,05) (Ribeiro, 2018; Marques et al., 2019b);
- SNP rs412263261 genotypes significantly milk production at 150 days of lactation (P150d; P<0.01), total milk production (PTotal; P<0.05) (Ribeiro, 2018; Marques et al., 2019a);
- SNP rs408430940 genotypes affected R (P<0,05) (Marques et al., 2019b);
- SNP rs406266481 genotypes significantly influenced Ptotal (P<0.01), lactation length (Dlact; P<0.01), fat free total solids content (TSTiMG; P<0.05) (Ribeiro, 2018; Marques et al., 2019a);
- the SNP rs604784916 genotypes significantly influenced Ptotal (P<0.05) and Dlact (P<0.05) (Ribeiro, 2018; Marques et al., 2019a);
- SNP rs600947105 genotypes significantly influenced TP (P<0.05), pH (P<0.05), gel firmness after 20 minutes (A20; P<0.01), and AR (P<0.01) (Ribeiro, 2018; Marques et al., 2019a);
- SNP rs401028781 genotypes tended to influenced milk fat production throughout lactation (P<0.10) (Sobreira, 2019);
- SNPs rs401028781 / rs412597723 haplotypes significantly affected TST (P<0.05) (Sobreira, 2019);
- SNP rs400524445 genotypes tended to affect fat and total solids contents (P<0,10) (Sobreira, 2019);
- SNP rs1094731965 genotypes significantly affected fat and total solids yields (P<0.05) and the evolution of milk urea-N concentration throughout lactation(P<0.05) (Sobreira, 2019).

If this methodology is applied to breed selection programmes, it is possible to determine the frequency of each allele in the population, estimate their effects on production parameters and use the more favourable SNPs as genetic markers. Moreover, it is a fast, reliable and affordable method to genotype specific SNPs linked to phenotypic traits for milk in sheep.

### Brief description of drawings

For easier understanding of this application, figures are attached in the annex that represent the preferred forms of implementation which nevertheless are not intended to limit the technique disclosed herein.

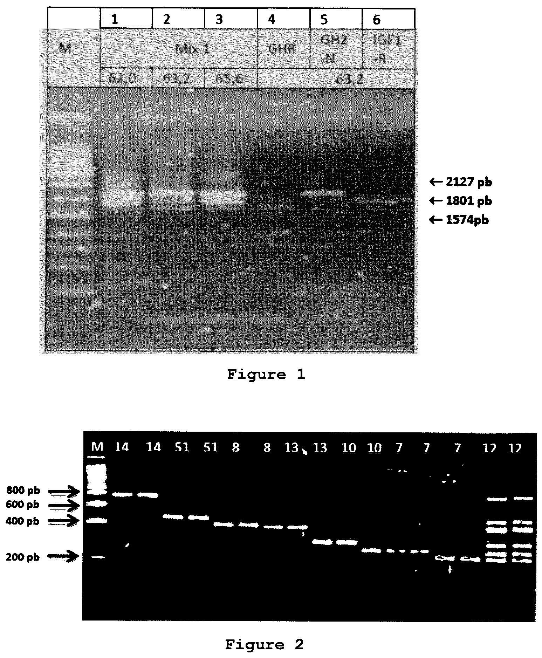

Figure 1 shows an 1% agarose gel electrophoresis of the fragments containing the GH2-N copy of the GH gene, exon 10 of the GHR gene and exon 8 of the IGF1-R gene amplified by multiplex PCR. M: NZYDNA Ladder III molecular mass marker (NZYTech, Lda., Lisbon, Portugal); 1-3: Mix 1 containing the three fragments under analysis amplified respectively at 62.0, 63.2 and 65.6 °C; 4: fragment containing exon 10 of the GHR gene (1574 bp); 5: fragment containing the GH2-N copy of the GH gene (2127 bp); 6: fragment containing exon 8 of the IGF1-R gene (1801 bp).
Figure 2 - Visualization of the 2% agarose gel electrophoresis of the PRL and PRLR gene fragments amplified by multiplex PCR. M: NZYDNA Ladder VIII molecular mass marker (NZYTech, Lda., Lisbon, Portugal); Bands marked 14 correspond to the fragment located in exon 10 of the PRLR gene (737 bp); 51: fragment containing exon 1 of the PRL gene (453 bp); 8: fragment containing exon 3 of the PRL gene (410 bp); 13: fragment 6 of the PRLR gene; 10: fragment containing the 5'UTR region of the PRL gene (304 bp); 7: fragment containing exon 2 of the PRL gene (264 bp); 12: fragment 4 of the PRLR gene; S13 and S14: all the previous fragments.

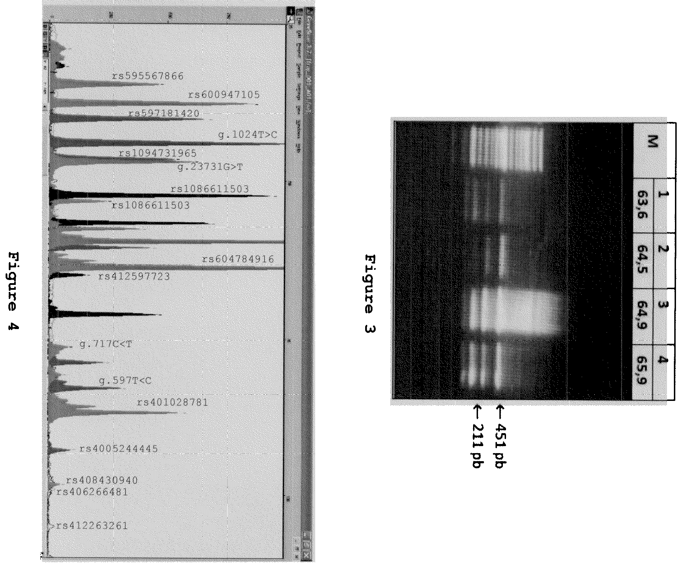

Figure 3 - Visualization by 2% agarose gel electrophoresis of the fragments containing part of the 5'-UTR region of the IGF-1 gene and exon 8 of the IGF1-R gene amplified by Multiplex PCR. M: NZYDNA Ladder VI molecular mass marker (NZYTech, Lda., Lisbon, Portugal); 1-4: Mix 3 containing part of the 5'-UTR region of the IGF-1 gene (451 bp) and exon 8 of the IGF1-R gene (211 bp) (and two other fragments not covered by this invention) amplified respectively at 63.6, 64.5, 64.9 and 65.9 °C.
Figure 4 - Example of an electropherogram relating to the genotyping of SNPs in the GH, GHR, PRL, PRLR, IGF1 and IGF1-R genes. The numbers in the electropherogram correspond to the SNPs No (see table 1).

### Detailed description of embodiments

Now, preferred embodiments of the present application will be described in detail with reference to the annexed drawings. However, they are not intended to limit the scope of this application.

The present invention relates to a method suitable for identifying 16 genetic markers, which are SNPs distributed across six somatotropic axis genes: GH, GHR, PRL, PRLR, IGF1 and IGF1-R, associated with the production and quality of sheep milk.

The SNPs to be identified is at least one SNP selected from the list comprising g.597T>C, g.717C>T, g.1024T>C in the GH gene; rs1086611503, rs595567866, rs597181420 in the GH receptor gene; rs400524445, rs1094731965 in the IGF1 receptor gene; g.23731G>T, rs412263261, rs408430940, rs406266481 in the Prolactin gene; rs604784916, rs600947105 in the PRL receptor gene; rs401028781, rs412597723 in the IGF1 gene.

The oligonucleotide primer sequences required to amplify the target gene fragments by polymerase chain reaction (PCR), and to perform the extension that leads to the identification of said SNPs via a SNaPshot method, are also provided within the scope of this invention in Tables 2A and 2B and Tables 3A and 3B.

**Table 2A. Oligonucleotide DNA primers for PCR amplification of the 10 gene fragments containing the 16 SNPs of the GH, GHR, PRL, PRLR, IGF1 and IGF1-R genes to be genotyped.**

| **SEQ ID NO.** | **Gene** | **Fragment** | **Oligonucleotide DNA primer** | | |
|---|---|---|---|---|---|
| | | | **Name** | **Size (bp)** | **Sequence (5'-3')** |
| ***1*** | *GH* | GH2-N | GH2N-F | 24 | agctgagaccctgtatgcacagcc |
| ***2*** | | | GH2N-R | 23 | gcccacagcacccctgctattgt |
| ***3*** | *GHR* | Exon 10 | GHR-F | 30 | tgcattcaatcttcctgaactgcaaa agcc |
| ***4*** | | | GHR-R | 35 | tggataaacaaggtccttctgtatag cacagagaa |
| ***5*** | *IGF1-R* | Exon 8 | IFG-1F_ex8 | 21 | cccaggtgaaagtgacgtcct |
| ***6*** | | | IFG-1R_ex8 | 21 | gtaaacggcgtactgcgtcca |
| ***7*** | *PRL* | 5'UTR | PRL-5uF | 25 | gaatcctctaacatgcattgccgtc |
| ***8*** | | | PRL-5uR | 23 | ggcaagtggaacttttgagccca |
| ***9*** | | Exon 1 | PRL-mF | 25 | ttcttccattcaagaaccactgtcc |
| ***10*** | | | PRL-mR | 25 | cctacatgttcatttctggtcagca |
| ***11*** | | Exon 2 | PRL-1F | 21 | tcgaagggacacctggcagtt |
| ***12*** | | | PRL-1R | 24 | acaatggaaggtattgcttcccca |
| ***13*** | | Exon 3 | PRL-2F | 24 | tcacctttctctgcatgtccagtt |
| ***14*** | | | PRL-1R | 25 | ggaatttagatgacaagcaactgtt |
| ***15*** | | Exon 10 | PRLR-3F | 22 | agtgaggaccaacacctgatgc |
| ***16*** | | | PRLR-3R | 21 | ttttgcgcttgcagatccggt |
| ***17*** | *IGF1* | 5'-UTR | IGF-1-F | 25 | acatctgctaatacaccttacccgt |
| ***18*** | | | IGF-1-R | 25 | ggaaaatcatttgcctctcagatgc |
| ***19*** | *IGF1-R* | Exon 3 | IFG-1F_ex3 | 24 | actatagtggctctccagttcacc |
| ***20*** | | | IFG1R_ex3 | 25 | tcacatgcaatcttgaacatgccac |

**Table 2B. Continuation of Table 2A - Oligonucleotide primers for PCR amplification of the 10 gene fragments containing the 16 SNPs of the GH, GHR, PRL, PRLR, IGF1 and IGF1-R genes to be genotyped.**

| **SEQ ID No.** | **[Conc.] µM** | **Amplicon size (bp)** | **Tm (°C)** | **Mix** |
|---|---|---|---|---|
| ***1*** | 1 | 2127 | 63.2 | 1 |
| ***2*** | | | | |
| ***3*** | 1 | 1574 | | |
| ***4*** | | | | |
| ***5*** | 0.6 | 1801 | | |
| ***6*** | | | | |
| ***7*** | 1 | 304 | 63 | 2 |
| ***8*** | | | | |
| ***9*** | | 453 | | |
| ***10*** | | | | |
| ***11*** | | 264 | | |
| ***12*** | | | | |
| ***13*** | | 410 | | |
| ***14*** | | | | |
| ***15*** | | 737 | | |
| ***16*** | | | | |
| ***17*** | 0.6 | 451 | 65.9 | 3 |
| ***18*** | | | | |
| ***19*** | | 211 | | |
| ***20*** | | | | |

The aforementioned oligonucleotide primers are suitable to enable DNA amplification via PCR in a single tube - multiplexing.

In one embodiment, the DNA sample comprises at least one gene from the group of GH (NCBI Gene ID 443329), GHR (NCBI Gene ID 443333), PRL (NCBI Gene ID 443317), PRLR (NCBI Gene ID 554331), IGF1 (NCBI Gene ID 443318) or IGF1-R (NCBI Gene ID 443515) genes.

In order to genotype the 16 SNPs targeted by this invention, it is necessary to amplify the DNA fragments containing these SNPs by PCR. Three multiplex PCR protocols have been established for this purpose:
**PCR-Multiplex 1** - Amplification of the copy GH2-N of GH, the exon 10 of GHR and the exon 8 of IFG1-R gene fragments by multiplex PCR using 6 primers comprising SEQ ID No. 1 to 6;
**Multiplex PCR 2** - Amplification of the 5'-UTR region, exon 1, exon 2 and exon 3 of PRL and exon 10 of PRLR gene fragments by multiplex PCR using 10 primers comprising SEQ ID No. 7 to 16;
**PCR-Multiplex 3** - Amplification of the 5'-UTR region of IGF1 and exon 3 of IGF1-R gene fragments by multiplex PCR using 4 primers comprising SEQ ID No. 17 to 20.

After multiplex PCR amplification of the desired fragments as mentioned above, the 16 SNPs are simultaneously genotyped using the single-base extension method and the set of oligonucleotide primers disclosed in Table 3A and Table 3B.

In one embodiment, this step is performed using a SNaPshot kit.

**Table 3A. Oligonucleotides DNA primers for the genotyping of 16 SNPs in the GH, GHR, PRL, PRLR, IGF1 and IGF1-R genes by a SNapShot method.**

| **SEQ ID NO.** | **Gene** | **Location** | **SNP** | **Sequence** | **SNP position** | **Allele** | **Δ aa** |
|---|---|---|---|---|---|---|---|
| **21** | *GH* | Exon 2 | g.597T>C | X12546 | 597 | T>C | Leu11P ro |
| **22** | | | g.717C>T | | 717 | C>T | Ala51V al |
| **23** | | Exon 3 | g.1024T>C | | 1024 | T>C | Phe78L eu |
| **24** | *GHR* | Exon 10 | rs1086611 503 | NC_019473.1 | 31835095 | C>T | Ser380 Pro |
| **25** | | | rs5955678 66 | | 31835059 | C>T | Glu392 Lys |
| **26** | | | rs5971814 20 | | 31834648 | C>T | Ala529 Thr |
| **27** | *PRL* | 5'UTR | g.23731G> T | AMGL02030943. 1 | 23731 | G>T | - |
| **28** | | Exon 1 | rs4122632 61 | NC_019477.1 | 34266402 | A>C | Val7Gl y |
| **29** | | Exon 2 | rs4084309 40 | | 34263597 | A>G | Thr33= |
| **30** | | Exon 3 | rs4062664 81 | | 34261223 | T>G | Arg75= |
| **31** | *PRLR* | Exon 10 | rs6047849 16 | NC_019473.1 | 39027431 | C>T | Arg351 Trp |
| **32** | | | rs6009471 05 | | 39027963 | C>T | Pro528 Leu |
| **33** | *IGF1* | 5'-UTR | rs4010287 81 | NC_019460.1 | 17132840 0 | C>G | - |
| **34** | | | rs4125977 23 | | 17132857 5 | G>A | - |
| **35** | *IGF1 -R* | Exon 3 | rs4005244 45 | NC_019475.1 | 7162694 | G>A | Gly74A rg |
| **36** | | Exon 8 | rs1094731 965 | | 7178705 | G>A | Met534 Arg |

**Table 3B. Continuation of Table 3A - Oligonucleotides DNA primers for the genotyping of 16 SNPs in the GH, GHR, PRL, PRLR, IGF1 and IGF1-R gene fragments by a SNapShot method.**

| **SEQ ID No.** | **Oligonucleotide primers** | | | |
|---|---|---|---|---|
| | **Sequence** | **Size (bp)** | **Direction** | **[Conc.]** |
| | | | | **µM** |
| **21** | (T)₅₉CAGGCAGAGCAGGGTGAAAGCCAGG | 84 | Fwd | 2.5 |
| **22** | (T)₅₀GCTTACAAACTCTTTGAAGGTGTCAGCA | 78 | Rev | 2.5 |
| **23** | (T)₁₀GGCTGGGATGGTTTCGGAGAAGCAGA | 36 | Rev | 2.5 |
| **24** | (T)₂₃TATCTTTGGGGCAAAGGATGATGAC | 48 | Rev | 1.5 |
| **25** | CAGCTGTTATGAACCTGACATTCTG | 25 | Fwd | 2.5 |
| **26** | (T)₅TTGGCATCTACCTCGCAGAAGTAAG | 30 | Fwd | 2.5 |
| **27** | (T)₁₆TTTGTGTAACCTTATCCTTAAGGAAA | 42 | Rev | 2.5 |
| **28** | (T)₈₃GCTGTACATACCTTTCTGCGCTGAA | 108 | Fwd | 7.5 |
| **29** | (T)₇₆CTCTTGTGTCAGGGTGTGGTCTCCAC | 102 | Rev | 12.5 |
| **30** | (T)₈₃ATGAACCCTTTGCCCTGGGCATACC | 108 | Fwd | 1.5 |
| **31** | (T)₄₁AGCGAAGGGCTGTCACAGCTGCCCC | 66 | Rev | 1.5 |
| **32** | (T)₅GGCGTCAACCTTCTCATTTTGTTTT | 30 | Rev | 1.5 |
| **33** | (C)₆₅GCAACCAGGACGAGGGGTCATCCCA | 90 | Rev | 2.5 |
| **34** | (C)₄₁GAATAGAGGAAGGAAGAAAGAGATT | 66 | Rev | 2.5 |
| **35** | (C)₇₁AAATTGACCCAGCGTCCTGCAGGTC | 96 | Fwd | 2.5 |
| **36** | (C)₁₇GTTGGGCGGGAGGTCCACGTCCACC | 42 | Rev | 2.5 |

The method of genotyping single nucleotide polymorphisms in somatotropic axis genes that are associated with the production and quality of sheep milk comprising the following steps:
- Extracting genomic DNA from a biological sample obtained from sheep;
- Amplifying the aforementioned DNA by multiplex PCR comprising:
   - A first multiplex PCR comprising the amplification of the copy GH2-N of the growth hormone, exon 10 of the growth hormone receptor and exon 8 of the insulin-like growth factor 1 receptor gene fragments using the primers comprising SEQ ID No. 1 to 6;
   - A second multiplex PCR comprising the amplification of the 5'-UTR region, exon 1, 2 and 3 of prolactin, and the exon 10 of the prolactin receptor gene fragments using the primers comprising SEQ ID No. 7 to 16;
   - A third multiplex PCR comprising the amplification of the 5'-UTR region of the insulin-like growth factor 1 and the exon 3 of the insulin-like growth factor 1 receptor gene fragments using the primers comprising SEQ ID No. 17 to 20;
- Preparing a sample comprising the products of the three multiplex PCR;
- Enzymatically purifying the sample with exonuclease I and shrimp alkaline phosphatase;
- Performing an extension reaction on the sample using the primers comprising SEQ ID No. 21 to 36;
- Adding shrimp alkaline phosphatase, purifying the extension reaction product and performing an enzymatic inactivation;
- Analysing an interpreting the results to obtain the genotypes of the SNPs.

In one embodiment, the biological sample is collected after the sheep's birth or throughout the live of the sheep. In one embodiment, the biological sample is selected from blood, milk, semen, or tissue collected from a sheep. In one embodiment, the biological sample is collected from embryo before transferring it to a surrogate.

In one embodiment, the first multiplex PCR is carried out with 1U of Taq DNA polymerase, 1 µM of each primer and 10 ng/µL of DNA sample. The enzyme activation is carried out for 2 minutes at 94°C, followed by 30 cycles of DNA denaturation for 10 seconds at 94°C, primer annealing for 20 seconds at 63.2°C, an extension for 1 minute at 72°C, and a final extension for 10 minutes at 72°C.

In one embodiment, the second multiplex PCR is carried out with 0.625 U of DFS-Taq DNA Polymerase, 1 µM of each primer and 5 ng/µL of DNA sample. The enzyme activation is carried out for 2 minutes at 94°C, followed by 30 cycles of DNA denaturation for 10 seconds at 94°C, primer annealing for 20 seconds at 63°C, an extension for 1 minute at 72°C, and a final extension for 10 minutes at 72°C.

In one embodiment, the third multiplex PCR is carried out with 1 U of DNA Polymerase, 0.6 µM of each primer, 2 mM of MgCl₂ and 10 ng/µL of DNA sample. The enzyme activation is carried out for 15 minutes at 95°C, followed by 30 cycles of DNA denaturation for 30 seconds at 95°C, primer annealing for 2 minutes at 65.9°C, an extension for 2 minute at 72°C, and a final extension for 10 minutes at 72°C.

In one embodiment, the products of the three multiplex PCR are combined in a ratio of 1:1.

In one embodiment, the first purification step of the sample is carried out for 15 minutes at 37°C, followed by 15 minutes at 80°C for enzymatic inactivation. During the purification step, exonuclease I eliminates the primers' excess, and shrimp alkaline phosphatase removes non incorporated dNTPs.

In one embodiment, the extension reaction is carried out with 25 cycles of 90°C for 10 seconds, 50°C for 5 seconds, 60°C for 3 seconds and a final extension step at 72°C for 2 minutes.

In one embodiment, the purification of the extension reaction product is carried out for 1 hour at 37°C, followed by 15 minutes at 75°C for enzymatic inactivation. In one embodiment, the extension reaction on the sample is carried out using a Snapshot multiplex kit.

In one embodiment, the resulting products are analyzed by capillary electrophoresis in an ABI3100 DNA genetic analyzer (Applied Biosystems) using the "GeneScan 120-Liz" (Applied Biosystems) as internal fragment size standard.

In one embodiment, the results can be analyzed and interpreted using GeneScan^{®} 3.7 and Genotyper^{®} 3.7 software respectively, to obtain the genotypes of the SNPs.

Here is also disclosed a kit for genotyping single nucleotide polymorphisms in somatotropic axis genes that are associated with the production and quality of sheep milk, wherein the kit comprises at least one oligonucleotide primer selected from the list comprising SEQ ID No. 1 to 36. The single nucleotide polymorphisms is at least one selected from the list comprising: g.597T>C, g.717C>T, g.1024T>C in the GH gene; rs1086611503, rs595567866, rs597181420 in the GH receptor gene; rs400524445, rs1094731965 in the IGF1 receptor gene; g.23731G>T, rs412263261, rs408430940, rs406266481 in the Prolactin gene; rs604784916, rs600947105 in the PRL receptor gene; rs401028781, rs412597723 in the IGF1 gene.

The methodology herein described makes it possible to simultaneously genotype the 16 SNPs described in Table 1 (see Figure 4) in sheep. This methodology has high accuracy and repeatability of results as long as the quality of the DNA is suitable for the procedure, saving time in terms of the analytical procedure (genotyping 16 specific SNPs that have previously been associated with parameters linked to the production and quality of milk and its technological suitability for cheese making).

Once the parameters described herein have been established, the methodology is straightforward to apply and does not require much optimisation, even if the DNA is obtained from tissues other than blood or from different sheep breeds. It also allows the genotypes of 96 animals to be obtained simultaneously, and the analysis of the results is easy to automate.

The SNPs proposed herein are polymorphic in the Assaf sheep breed. As this is a technique that allows for sensitive allele-frequency detection, its usefulness in selecting animals with a specific allele is an added value when implementing marker-assisted selection (MAS), in this case, based on the described SNPs as genetic markers.

For example, if a breeder wants to keep animals on the farm that have the best aptitude for milk production and at the same time a good aptitude for cheese production, the farmer can select an animal with that specific genotype obtained with this method.

However, this patent application is not about which genotype should be selected for a particular production trait. It is simply a tool for quickly and practically obtaining the genotypes of animals, enabling the genetic characterization of the population and the ranking of the selected animals against the genetic value of animals with the same known genotypes and phenotypes for the traits of interest.

In one embodiment, the genomic DNA samples were obtained from blood of Assaf sheep breed for the purpose of the experimental data herein disclosed.

For experimental results shown in Figures 1 to 4, blood samples (9 ml) were obtained by jugular venepuncture on potassium-ethylenediaminetetracetic acid (EDTA; final concentration of 1.6 mg/ml blood). DNA extraction was performed using a phenol/chloroform free method ("NucleoSpin^{®} Blood" (Macherey-Nagel GmbH & Co. KG)) following the manufacturer's instructions.

### Example

### PCR-Multiplex 1 - Amplification of the GH (copy GH2-N), GHR (exon 10) and IFG1-R (exon 8) gene fragments.

Amplification of the fragments containing the GH2-N copy of the GH gene (2127 bp), exon 10 of the GHR gene (1574 bp) and exon 8 of the IGF1-R gene (1801 bp) with the "NZYTaq II 2x Green Master Mix" (NZYTech Genes & Enzymes, Lisbon, Portugal) was carried out in a final reaction volume of 12.5 µl (Mix1), considering the following concentrations: 1U of enzyme; 1.0 µM of each of the six oligonucleotide primers described in Table 2 comprising SEQ ID No. 1 to 6 and 10 ng/µl of DNA. During the reaction, carried out in a thermal cycler, the enzyme was activated at 94 °C for 2 min, followed by 30 cycles of DNA denaturation at 94 °C for 10 s, oligonucleotide ligation at 63.2 °C for 20 s and extension at 72 °C for 1 min, and then a final extension at 72 °C for 10 min.

The specificity of the multiplex PCR reaction was validated by electrophoresing the samples (5 V.cm⁻¹) on a 1% agarose gel in 1x TBE buffer (0.09M borate, 0.02 M EDTA).

The samples stained with ethidium bromide were visualized under UV light using a transilluminator (BioDoc-ItTM Imaging System) and showed good yields and specificity as can be seen in Figure 1, which supports the validity of the oligonucleotide primers for amplifying the target fragments of Mix 1 as disclosed in this invention.

### Multiplex PCR 2 - Amplification of PRL (5'-UTR region, exon 1, 2 and 3) and PRLR (exon 10) gene fragments by multiplex PCR.

Amplification of the fragments containing the 5'-UTR region (304 bp), exon 1 (453 bp), 2 (264 bp) and 3 (410 bp) of the PRL gene, and exon 10 of the PRLR gene (737 bp) with "DFS-Taq DNA Polymerase" (BIORON GmbH - Ludwigshafen - Germany) was carried out in a final reaction volume of 12.5 µl (Mix2), considering the following concentrations: 0.625 U of enzyme; 1.0 µM of each of the ten oligonucleotide primers described in Table 2 comprising SEQ ID No. 7 to 16 and 5 ng/µl of DNA. During the reaction, carried out in a thermal cycler, activation of the enzyme was run at 94 °C for 2 min, followed by 30 cycles of DNA denaturation at 94 °C for 10 s, oligonucleotide ligation at 63 °C for 20 s and extension at 72 °C for 1 min, and then a final extension at 72 °C for 10 min.

The specificity of the multiplex PCR reaction was validated by electrophoresing the samples (5 V.cm⁻¹) on a 2% agarose gel in 1x TBE buffer (0.09M borate, 0.02 M EDTA). The samples stained with ethidium bromide were visualized under UV light using a transilluminator (BioDoc-ItTM Imaging System) and showed good yields and specificity as can be seen in Figure 2, which supports the validity of the oligonucleotide primers for amplifying the target fragments of Mix 2 as claimed in this invention.

### PCR-Multiplex 3 - Amplification of the IGF1 (5'-UTR region) and IGF1-R (exon 3) gene fragments.

Amplification of the fragments containing part of the 5'-UTR region of the IGF-1 gene (451 bp) and exon 3 of the IGF-1R gene (211 bp) with the "5x HOT FIREPol Multiplex Mix" (Solis BIODYne, Tartu, Estonia) was carried out in a final reaction volume of 12.5 µl (Mix 3), considering the following concentrations: 1x enzyme; 0.6 µM of each of the four oligonucleotide primers described in Table 2 comprising SEQ ID No. 17 to 20; 2 mM MgCl₂ and 10 ng/µl of DNA. During the reaction, carried out in a thermal cycler, the enzyme was activated at 95 °C for 15 min, followed by 30 cycles of DNA denaturation at 95 °C for 30 s, oligonucleotide ligation at 65.9 °C for 2:00 min and extension at 72 °C for 2 min, and finally a final extension at 72 °C for 10 min.

The specificity of the multiplex PCR reaction was validated by electrophoresing the samples (5 V.cm⁻¹) on a 2% agarose gel in 1x TBE buffer (0.09M borate, 0.02 M EDTA). The samples stained with ethidium bromide were visualized under UV light using a transilluminator (BioDoc-ItTM Imaging System) and showed good yields and specificity as can be seen in Figure 3, which supports the validity of the oligonucleotide primers for amplifying the target fragments of Mix 3 as claimed in this invention.

The results were analyzed and interpreted using the GeneScan^{®} 3.7 software and the Genotyper^{®} 3.7 program, respectively, to obtain the genotypes of the SNPs analyzed.

### Genotyping by SNaPshot analysis:

After PCR amplification of the desired fragments, the 16 proposed SNPs were simultaneously genotyped using the single base extension method, using the "ABI PRISM SNaPshotTM Multiplex" kit (Applied Biosystems, California, USA).

The proposed experimental procedure was as follows:
- Formation of a composite sample containing 2 µl of each PCR product obtained according to protocols 1 to 3 above;
- Cleaning of 6 µl of the composite sample of PCR products with 2.4 µl PCR ExoSap-IT^{®} (USB Product Affymetrix, Inc.) (or a simulant reagent), according to the manufacturer's instructions (15 min. at 37°C, followed by 15 min. at 80°C for enzymatic inactivation).
- After purifying the samples, the extension reaction was carried out in a final volume of 12.4 µl, adding 1 µl of the mix containing the oligonucleotide primers described in Table 3A and 3B comprising SEQ ID No. 21 to 36, 8.4 µl of purified PCR product and 3 µl of "SNaPshot Multiplex Ready Reaction Mix" (Applied Biosystems, UK), under the following conditions: 25 cycles of 96 °C for 10 s, 50 °C for 5 s, 60 °C for 30 s and a final extension at 72 °C for 2 minutes.
- Next, 2 µl of SAP (Shrimp Alkaline Phosphatase, Affymetrix, Inc.) was added to the product of the extension reaction to clean the reaction products at 37 °C for 1 hour, followed by 75 °C for 15 minutes for enzyme inactivation.
- The samples were analyzed by capillary electrophoresis with an ABI3100 DNA genetic analyzer (Applied Biosystems) (or similar) using the "GeneScan 120-Liz" (Applied Biosystems) with internal fragment size standard.

Figures 4 shows evidence of the suitability of the methodology described for identifying the SNPs covered by the present invention.

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

### Bibliography

Akers, R.M., Ellis, S.E., Berry, S.D., 2005. Ovarian and IGF-I axis control of mammary development in prepubertal heifers. Domestic Animal Endocrinology 29, 259-267. https://doi.org/10.1016/j.domaniend.2005.02.037
Bauman, D.E. 1999. Bovine somatotropin and lactation: from basic science to commercial application. Domestic Animal Endocrinology 17, 101-116. https://doi.org/10.1016/S0739-7240(99)00028-4
Boutinaud, M., Rousseau, C., Keisler, D.H., Jammes, H., 2003. Growth Hormone and Milking Frequency Act Differently on Goat Mammary Gland in Late Lactation. Journal of Dairy Science 86, 509-520. https://doi.org/10.3168/jds.S0022-0302(03)73629-7
Burgos, S.A., Cant, J.P., 2010. IGF-1 stimulates protein synthesis by enhanced signaling through mTORC1 in bovine mammary epithelial cells. Domestic Animal Endocrinology 38, 211-221. https://doi.org/10.1016/j.domaniend.2009.10.005
Capuco, A.V., Wood, D.L., Baldwin, R., Mcleod, K., Paape, M.J., 2001. Mammary Cell Number, Proliferation, and Apoptosis During a Bovine Lactation: Relation to Milk Production and Effect of bST1. Journal of Dairy Science 84, 2177-2187. https://doi.org/10.3168/jds.S0022-0302(01)74664-4
Carter-Su, C., Rui, L., Herrington, J., 2000. Role of the tyrosine kinase JAK2 in signal transduction by growth hormone. Pediatric Nephrology 14, 550-557. https://doi.org/10.1007/s004670000366
Clarkson, R.W.E., Boland, M.P., Kritikou, E.A., Lee, J.M., Freeman, T.C., Tiffen, P.G., Watson, C.J., 2006. The Genes Induced by Signal Transducer and Activators of Transcription (STAT)3 and STATS in Mammary Epithelial Cells Define the Roles of these STATs in Mammary Development. Molecular Endocrinology 20, 675-685. https://doi.org/10.1210/me.2005-0392
Dettori, M.L., Rocchigiani, A.M., Luridiana, S., Mura, M.C., Carcangiu, V., Pazzola, M., Vacca, G.M., 2013. Growth hormone gene variability and its effects on milk traits in primiparous Sarda goats. Journal of Dairy Research 80, 255-262. https://doi.org/10.1017/S0022029913000174
Etherton, T.D., Bauman, D.E., 1998. Biology of Somatotropin in Growth and Lactation of Domestic Animals. Physiological Reviews 78, 745-761. https://doi.org/10.1152/physrev.1998.78.3.745
Falaki, M., Prandi, A., Corradini, C., Sneyers, M., Gengler, N., Massart, S., Fazzini, U., Burny, A., Portetelle, D., Renaville, R., 1997. Relationships of growth hormone gene and milk protein polymorphisms to milk production traits in Simmental cattle. Journal of Dairy Research 64, 47-56. https://doi.org/10.1017/S0022029996001872
Gutiérrez-Gil, B., El-Zarei, M.F., Alvarez, L., Bayón, Y., De La Fuente, L.F., San Primitivo, F., Arranz, J.-J., 2009. Quantitative trait loci underlying milk production traits in sheep. Animal Genetics 40, 423-434. https://doi.org/10.1111/j.1365-2052.2009.01856.x
Fleming, J.M., Leibowitz, B.J., Kerr, D.E., Cohick, W.S. 2005. IGF-I differentially regulates IGF-binding protein expression in primary mammary fibroblasts and epithelial cells. Journal of Endocrinology 186, 165-178. https://doi.org/10.1677/joe.1.06164
Jonas, E., Thomson, P.C., Hall, E.J., McGill, D., Lam, M.K., Raadsma, H.W., 2011. Mapping quantitative trait loci (QTL) in sheep. IV. Analysis of lactation persistency and extended lactation traits in sheep. Genetics Selection Evolution 43, 22. https://doi.org/10.1186/1297-9686-43-22
Kann, G., Delobelle-Deroide, A., Belair, L., Gertler, A., Djiane, J., 1999. Demonstration of in vivo mammogenic and lactogenic effects of recombinant ovine placental lactogen and mammogenic effect of recombinant ovine GH in ewes during artificial induction of lactation. https://doi.org/10.1677/joe.0.1600365
Li, H.-M., Wang, C.-M., Li, Q.-Z., Gao, X.-J., 2012. MiR-15a Decreases Bovine Mammary Epithelial Cell Viability and Lactation and Regulates Growth Hormone Receptor Expression. Molecules 17. https://doi.org/10.3390/molecules171012037
Lucy, M.C., Hauser, S.D., Eppard, P.J., Krivi, G.G., Clark, J.H., Bauman, D.E., Collier, R.J., 1993. Variants of somatotropin in cattle: gene frequencies in major dairy breeds and associated milk production. Domestic Animal Endocrinology 10, 325-333. https://doi.org/10.1016/0739-7240(93)90036-B
Marques, M.R., Ribeiro, D.S., Gomes, S. Belo, A.T., Ribeiro, J.M.B., Martins, A.P.L., Belo, C.C. 2019b. Associations of single nucleotide polymorphisms in the ovine prolactin and prolactin receptor genes with milk traits in Assaf dairy sheep. 37th International Society for Animal Genetics Conference. ISAG 2019 Abstract Book pp.77.
Marques, M.R., Ribeiro, D.S., Gomes, S. Belo, A.T., Ribeiro, J.M.B., Martins, A.P.L., Belo, C.C. 2019a. Associations of single nucleotide polymorphisms in the ovine prolactin and prolactin receptor genes with milk traits in Assaf dairy sheep. 37th International Society for Animal Genetics Conference. ISAG 2019 Abstract Book pp.77-78.
Marques, M.R., Ribeiro, J.R.B., Belo, A.T., Gomes, S., Martins, A.P.L., Belo, C.C. 2019c. Effects of nonsynonymous SNPs at GH2-N and GHR genes on coagulation properties of Assaf ewes' milk. 70th Annual Meeting of the European Federation of Animal Science. Book of Abstracts No. 25, pág. 224.
Marques, M.R., Santos, I.C., Carolino, N., Belo, C.C., Renaville, R., Cravador, A., 2006. Effects of genetic polymorphisms at the growth hormone gene on milk yield in Serra da Estrela sheep. Journal of Dairy Research 73, 394-405. https://doi.org/10.1017/S0022029906001932
Martins, A.P.L., Belo, A.T., Vasconcelos, M.M., Fontes, A.L., Pereira, E.A., Belo, C.C., 2009. Characterisation of production system of Niza cheese (PDO): Effect of sheep breed on milk composition and coagulation properties. Changes in sheep and goat farming systems at the beginning of the 21st century: research, tools, methods and initiatives in favour of a sustainable development. Options Méditerranéennes, Série A, Séminaires Méditerranéens 91, 221-225.
Mateescu, R.G., Thonney, M.L., 2010. Genetic mapping of quantitative trait loci for milk production in sheep. Anim Genet 41. https://doi.org/10.1111/j.1365-2052.2010.02045.x
Modha, G., Blanchard, A., Iwasiow, B., Juan Mao, X., Troup, S., Adeyinka, A., Watson, P., Shiu, R., Myal, Y., 2004. Developmental Changes in Insulin-like Growth Factor I Receptor Gene Expression in the Mouse Mammary Gland. null 30, 127-140. https://doi.org/10.1081/ERC-120029892
Pisera, D., Theas, S., De Laurentiis, A., Lasaga, M., Duvilanski, B., Seilicovich, A., 1998. The hormonal status modulates the effect of neurokinin A on prolactin secretion in female rats. Journal of Endocrinology 159, 389-395. https://doi.org/10.1677/joe.0.1590389
Pryce, J.E., Haile-Mariam, M., Verbyla, K., Bowman, P.J., Goddard, M.E., Hayes, B.J., 2010. Genetic markers for lactation persistency in primiparous Australian dairy cows. Journal of Dairy Science 93, 2202-2214. https://doi.org/10.3168/jds.2009-2666
Ribeiro, D.S. 2018. Identificação de SNPs dos genes da prolactina e do receptor da prolactina e a sua associação com aprodução e a qualidade do leite em ovelhas Assaf.
Rosen, J.M., Wyszomierski, S.L., Hadsell, D., 1999. REGULATION OF MILK PROTEIN GENE EXPRESSION. Annu. Rev. Nutr. 19, 407-436. https://doi.org/10.1146/annurev.nutr.19.1.407
Sakamoto, K., Komatsu, T., Kobayashi, T., Rose, M.T., Aso, H., Hagino, A., Obara, Y., 2005. Growth hormone acts on the synthesis and secretion of α-casein in bovine mammary epithelial cells. Journal of Dairy Research 72, 264-270. https://doi.org/10.1017/S0022029905000889
Sciascia, Q., Pacheco, D., McCoard, S.A., 2013. Increased milk protein synthesis in response to exogenous growth hormone is associated with changes in mechanistic (mammalian) target of rapamycin (mTOR)C1-dependent and independent cell signaling. Journal of Dairy Science 96, 2327-2338. https://doi.org/10.3168/jds.2012-6267
Sobreira, S. 2019. Efeito de cinco SNPs dos genes do IGF-1 e do seu receptor sobre a produção e a qualidade do leite em ovelhas Assaf.
Vacca, G.M., Dettori, M.L., Balia, F., Luridiana, S., Mura, M.C., Carcangiu, V., Pazzola, M., 2013. Sequence polymorphisms at the growth hormone GH1/GH2-N and GH2-Z gene copies and their relationship with dairy traits in domestic sheep (Ovis aries). Molecular Biology Reports 40, 5285-5294. https://doi.org/10.1007/s11033-013-2629-9

## Claims

1. A method of genotyping single nucleotide polymorphisms in somatotropic axis genes that are associated with the production and quality of sheep milk comprising the following steps:
- Extracting genomic DNA from a biological sample obtained from sheep;
- Amplifying the aforementioned DNA by multiplex PCR comprising:
- A first multiplex PCR comprising the amplification of the copy GH2-N of the growth hormone, exon 10 of the growth hormone receptor and exon 8 of the insulin-like growth factor 1 receptor gene fragments using the primers comprising SEQ ID No. 1 to 6;
- A second multiplex PCR comprising the amplification of the 5'-UTR region, exon 1, 2 and 3 of prolactin, and the exon 10 of the prolactin receptor gene fragments using the primers comprising SEQ ID No. 7 to 16;
- A third multiplex PCR comprising the amplification of the 5'-UTR region of the insulin-like growth factor 1 and the exon 3 of the insulin-like growth factor 1 receptor gene fragments using the primers comprising SEQ ID No. 17 to 20;
- Preparing a sample comprising the products of the three multiplex PCR;
- Enzymatically purifying the sample with exonuclease I and shrimp alkaline phosphatase;
- Performing an extension reaction on the sample using the primers comprising SEQ ID No. 21 to 36;
- Adding shrimp alkaline phosphatase, purifying the extension reaction product and performing an enzymatic inactivation;
- Analysing an interpreting the results to obtain the genotypes of the SNPs.

2. Method according to the previous claim, wherein the biological sample is collected after the sheep's birth or throughout the live of the sheep.

3. Method according to any of the previous claims, wherein the biological sample is selected from blood, milk, semen, or tissue collected from a sheep.

4. Method according to any of the previous claims, wherein the DNA sample comprises at least one gene from the group of GH (NCBI Gene ID 443329), GHR (NCBI Gene ID 443333), PRL (NCBI Gene ID 443317), PRLR (NCBI Gene ID 554331), IGF1 (NCBI Gene ID 443318) or IGF1-R (NCBI Gene ID 443515).

5. Method according to any of the previous claims, wherein the single nucleotide polymorphisms is at least one selected from the list comprising: g.597T>C, g.717C>T, g.1024T>C in the GH gene; rs1086611503, rs595567866, rs597181420 in the GH receptor gene; rs400524445, rs1094731965 in the IGF1 receptor gene; g.23731G>T, rs412263261, rs408430940, rs406266481 in the Prolactin gene; rs604784916, rs600947105 in the PRL receptor gene; rs401028781, rs412597723 in the IGF1 gene.

6. Method according to any of the previous claims, wherein the first multiplex PCR is carried out with 1U of Taq DNA polymerase, 1 µM of each primer and 10 ng/µL of DNA sample, the enzyme activation is carried out for 2 minutes at 94°C, followed by 30 cycles of DNA denaturation for 10 seconds at 94°C, primer annealing for 20 seconds at 63.2°C, an extension for 1 minute at 72°C, and a final extension for 10 minutes at 72°C.

7. Method according to any of the previous claims, wherein the second multiplex PCR is carried out with 0.625 U of DFS-Taq DNA Polymerase, 1 µM of each primer and 5 ng/µL of DNA sample, the enzyme activation is carried out for 2 minutes at 94°C, followed by 30 cycles of DNA denaturation for 10 seconds at 94°C, primer annealing for 20 seconds at 63°C, an extension for 1 minute at 72°C, and a final extension for 10 minutes at 72°C.

8. Method according to any of the previous claims, wherein the third multiplex PCR is carried out with 1 U of DNA Polymerase, 0.6 µM of each primer, 2 mM of MgCl₂ and 10 ng/µL of DNA sample, the enzyme activation is carried out for 15 minutes at 95°C, followed by 30 cycles of DNA denaturation for 30 seconds at 95°C, primer annealing for 2 minutes at 65.9°C, an extension for 2 minute at 72°C, and a final extension for 10 minutes at 72°C.

9. Method according to any of the previous claims, wherein the products of the three multiplex PCR are combined in a ratio of 1:1.

10. Method according to any of the previous claims, wherein the first purification step of the sample is carried out for 15 minutes at 37°C, followed by 15 minutes at 80°C for enzymatic inactivation.

11. Method according to any of the previous claims, wherein the extension reaction is carried out with 25 cycles of 90°C for 10 seconds, 50°C for 5 seconds, 60°C for 3 seconds and a final extension step at 72°C for 2 minutes.

12. Method according to any of the previous claims, wherein the purification of the extension reaction product is carried out for 1 hour at 37°C, followed by 15 minutes at 75°C for enzymatic inactivation.

13. Method according to any of the previous claims, wherein the resulting products are analyzed by capillary electrophoresis.

14. Kit for genotyping single nucleotide polymorphisms in somatotropic axis genes that are associated with the production and quality of sheep milk, wherein the kit comprises the primer set defined by SEQ ID No. 1 to 36 for use in the method disclosed in any of the claims 1 to 13.

15. Kit according to the previous claim, wherein the single nucleotide polymorphisms is at least one selected from the list comprising: g.597T>C, g.717C>T, g.1024T>C in the GH gene; rs1086611503, rs595567866, rs597181420 in the GH receptor gene; rs400524445, rs1094731965 in the IGF1 receptor gene; g.23731G>T, rs412263261, rs408430940, rs406266481 in the Prolactin gene; rs604784916, rs600947105 in the PRL receptor gene; rs401028781, rs412597723 in the IGF1 gene.

## Patentansprüche

1. Verfahren zur Genotypisierung von Einzelnukleotid-Polymorphismen in Genen der somatotropen Achse, die mit der Produktion und Qualität von Schafsmilch in Zusammenhang stehen, umfassend die folgenden Schritte:
- Extraktion genomischer DNA aus einer von Schafen gewonnenen biologischen Probe;
- Amplifikation der vorgenannten DNA mittels Multiplex-PCR, umfassend:
- eine erste Multiplex-PCR, umfassend die Amplifikation der Genfragmente der GH2-N-Kopie des Wachstumshormons, des Exons 10 des Wachstumshormonrezeptors und des Exons 8 des Insulinähnlichen Wachstumsfaktor-1-Rezeptors unter Verwendung von Primern, welche die Sequenzen gemäß SEQ ID Nr. 1 bis 6 umfassen;
- eine zweite Multiplex-PCR, welche die Amplifikation der 5'-UTR-Region, der Exons 1, 2 und 3 des Prolaktins sowie des Exons 10 des Prolaktinrezeptor-Gens unter Verwendung von Primern, welche die Sequenzen gemäß SEQ ID Nr. 7 bis 16 umfassen;
- eine dritte Multiplex-PCR, welche die Amplifikation der 5'-UTR-Region des insulinähnlichen Wachstumsfaktors 1 und des Exons 3 der Genfragmente des Rezeptors für den insulinähnlichen Wachstumsfaktor 1 unter Verwendung von Primern, welche die Sequenzen gemäß SEQ ID Nr. 17 bis 20 umfassen;
- Vorbereitung einer Probe, welche die Produkte der drei Multiplex-PCR-Reaktionen enthält;
- Enzymatische Reinigung der Probe mit Exonuklease I und Shrimp-Alkaline-Phosphatase;
- Durchführung einer Verlängerungsreaktion an der Probe unter Verwendung von Primern, welche die Sequenzen gemäß SEQ ID Nr. 21 bis 36 umfassen;
- Zugabe von Shrimp-Alkaline-Phosphatase, Reinigung des Verlängerungsreaktionsprodukts und Durchführung einer enzymatischen Inaktivierung;
- Analysieren und Auswerten der Ergebnisse, um die Genotypen der SNP zu ermitteln.

2. Verfahren nach dem vorstehenden Anspruch, wobei die biologische Probe nach der Geburt des Schafs oder im Laufe seines Lebens entnommen wird.

3. Verfahren nach einem der vorstehenden Ansprüche, wobei die biologische Probe aus Blut, Milch, Sperma oder Gewebe stammt, das einem Schaf entnommen wurde.

4. Verfahren nach einem der vorstehenden Ansprüche, wobei die DNA-Probe mindestens ein Gen aus der Gruppe bestehend aus GH (NCBI-Gen-ID 443329), GHR (NCBI-Gen-ID 443333), PRL (NCBI-Gen-ID 443317), PRLR (NCBI-Gen-ID 554331), IGF1 (NCBI-Gen-ID 443318) oder IGF1-R (NCBI-Gen-ID 443515) umfasst.

5. Verfahren nach einem der vorstehenden Ansprüche, wobei der Einzelnukleotid-Polymorphismus mindestens einer ist, der aus der Liste ausgewählt ist, die Folgendes umfasst: g.597T>C, g.717C>T, g.1024T>C im GH-Gen; rs1086611503, rs595567866, rs597181420 im GH-Rezeptor-Gen; rs400524445, rs1094731965 im IGF1-Rezeptor-Gen; g.23731G>T, rs412263261, rs408430940, rs406266481 im Prolaktin-Gen; rs604784916, rs600947105 im PRL-Rezeptor-Gen; rs401028781, rs412597723 im IGF1-Gen.

6. Verfahren nach einem der vorstehenden Ansprüche, wobei die erste Multiplex-PCR mit 1 U Taq-DNA-Polymerase, jeweils 1 µM jedes Primers und 10 ng/µL DNA-Probe durchgeführt wird, die Enzymaktivierung 2 Minuten lang bei 94 °C erfolgt, gefolgt von 30 Zyklen der DNA-Denaturierung für jeweils 10 Sekunden bei 94 °C, Primer-Anlagerung für 20 Sekunden bei 63,2 °C, einer Extension für 1 Minute bei 72 °C sowie einer abschließenden Extension für 10 Minuten bei 72 °C.

7. Verfahren gemäß einem der vorstehenden Ansprüche, wobei die zweite Multiplex-PCR mit 0,625 U DFS-Taq-DNA-Polymerase, jeweils 1 µM jedes Primers und 5 ng/µL DNA-Probe durchgeführt wird, die Enzymaktivierung 2 Minuten lang bei 94 °C erfolgt, gefolgt von 30 Zyklen der DNA-Denaturierung für 10 Sekunden bei 94 °C, Primer-Anlagerung für 20 Sekunden bei 63 °C, einer Extension für 1 Minute bei 72 °C sowie einer abschließenden Extension für 10 Minuten bei 72 °C.

8. Verfahren nach einem der vorstehenden Ansprüche, wobei die dritte Multiplex-PCR mit 1 U DNA-Polymerase, jeweils 0,6 pM jedes Primers, 2 mM MgCl2 und 10 ng/µL DNA-Probe durchgeführt wird, die Enzymaktivierung 15 Minuten lang bei 95 °C erfolgt, gefolgt von 30 Zyklen der DNA-Denaturierung für jeweils 30 Sekunden bei 95 °C, Primer-Annealing für 2 Minuten bei 65,9 °C, einer Extension für 2 Minuten bei 72 °C sowie einer abschließenden Extension für 10 Minuten bei 72 °C.

9. Verfahren nach einem der vorstehenden Ansprüche, wobei die Produkte der drei Multiplex-PCR-Reaktionen im Verhältnis 1:1 kombiniert werden.

10. Verfahren nach einem der vorstehenden Ansprüche, wobei der erste Reinigungsschritt der Probe 15 Minuten lang bei 37 °C durchgeführt wird, gefolgt von 15 Minuten bei 80 °C zur enzymatischen Inaktivierung.

11. Verfahren nach einem der vorstehenden Ansprüche, wobei die Verlängerungsreaktion mit 25 Zyklen bei 90 °C für 10 Sekunden, 50 °C für 5 Sekunden, 60 °C für 3 Sekunden und einem abschließenden Verlängerungsschritt bei 72 °C für 2 Minuten durchgeführt wird.

12. Verfahren nach einem der vorstehenden Ansprüche, wobei die Reinigung des Produkts der Verlängerungsreaktion 1 Stunde lang bei 37 °C durchgeführt wird, gefolgt von 15 Minuten bei 75 °C zur enzymatischen Inaktivierung.

13. Verfahren nach einem der vorstehenden Ansprüche, wobei die resultierenden Produkte mittels Kapillarelektrophorese analysiert werden.

14. Kit zur Genotypisierung von Einzelnukleotid-Polymorphismen in Genen der somatotropen Achse, die mit der Produktion und Qualität von Schafsmilch in Zusammenhang stehen, wobei das Kit den durch die SEQ ID Nr. 1 bis 36 definierten Primersatz zur Verwendung in dem in einem der Ansprüche 1 bis 13 offenbarten Verfahren umfasst.

15. Kit nach dem vorstehenden Anspruch, wobei der Einzelnukleotid-Polymorphismus mindestens einer ist, der aus der Liste ausgewählt ist, die Folgendes umfasst: g.597T>C, g.717C>T, g.1024T>C im GH-Gen; rs1086611503, rs595567866, rs597181420 im GH-Rezeptor-Gen; rs400524445, rs1094731965 im IGF1-Rezeptor-Gen; g.23731G>T, rs412263261, rs408430940, rs406266481 im Prolaktin-Gen; rs604784916, rs600947105 im PRL-Rezeptor-Gen; rs401028781, rs412597723 im IGF1-Gen.

## Revendications

1. Procédé de génotypage de polymorphismes mononucléotidiques dans des gènes de l'axe somatotrope qui sont associés à la production et à la qualité du lait ovin, comprenant les étapes suivantes :
- Extraire l'ADN génomique d'un échantillon biologique obtenu à partir d'un ovin ;
- Amplifier l'ADN susmentionné par PCR multiplex comprenant :
- Une première PCR multiplex comprenant l'amplification de la copie GH2-N de l'hormone de croissance, de l'exon 10 du récepteur de l'hormone de croissance et de l'exon 8 du récepteur du facteur de croissance analogue à l'insuline 1, en utilisant les amorces comprenant les SEQ ID No. 1 à 6 ;
- Une seconde PCR multiplex comprenant l'amplification des fragments géniques de la région 5'-UTR, des exons 1, 2 et 3 de la prolactine et de l'exon 10 du récepteur de la prolactine, en utilisant des amorces comprenant les SEQ ID No. 7 à 16 ;
- Une troisième PCR multiplex comprenant l'amplification des fragments géniques de la région 5'-UTR du facteur de croissance analogue à l'insuline 1 et de l'exon 3 du récepteur du facteur de croissance analogue à l'insuline 1, en utilisant des amorces comprenant les SEQ ID No. 17 à 20 ;
- Préparer un échantillon comprenant les produits des trois PCR multiplex ;
- Purifier l'échantillon par voie enzymatique avec de l'exonucléase I et de la phosphatase alcaline de crevette ;
- Effectuer une réaction d'extension sur l'échantillon en utilisant des amorces comprenant les SEQ ID No. 21 à 36 ;
- Ajouter de la phosphatase alcaline de crevette, purifier le produit de la réaction d'extension et effectuer une inactivation enzymatique ;
- Analyser et interpréter les résultats afin d'obtenir les génotypes des SNP.

2. Procédé selon la revendication précédente, dans lequel l'échantillon biologique est prélevé après la naissance de l'ovin ou tout au long de la vie de l'ovin.

3. Procédé selon l'une quelconque des revendications précédentes, dans lequel l'échantillon biologique est choisi parmi le sang, le lait, le sperme ou un tissu prélevé sur un ovin.

4. Procédé selon l'une quelconque des revendications précédentes, dans lequel l'échantillon d'ADN comprend au moins un gène du groupe constitué de GH (NCBI Gene ID 443329), GHR (NCBI Gene ID 443333), PRL (NCBI Gene ID 443317), PRLR (NCBI Gene ID 554331), IGF1 (NCBI Gene ID 443318) ou IGF1-R (NCBI Gene ID 443515).

5. Procédé selon l'une quelconque des revendications précédentes, dans lequel les polymorphismes mononucléotidiques sont au moins l'un des polymorphismes choisis dans la liste comprenant : g.597T>C, g.717C>T, g.1024T>C dans le gène GH ; rs1086611503, rs595567866, rs597181420 dans le gène du récepteur de GH ; rs400524445, rs1094731965 dans le gène du récepteur d'IGF1 ; g.23731G>T, rs412263261, rs408430940, rs406266481 dans le gène de la prolactine ; rs604784916, rs600947105 dans le gène du récepteur de PRL ; rs401028781, rs412597723 dans le gène IGF1.

6. Procédé selon l'une quelconque des revendications précédentes, dans lequel la première PCR multiplex est réalisée avec 1U d'ADN polymérase Taq, 1 µM de chaque amorce et 10 ng/µL d'échantillon d'ADN, l'activation de l'enzyme est effectuée pendant 2 minutes à 94 °C, suivie de 30 cycles de dénaturation de l'ADN pendant 10 secondes à 94 °C, d'hybridation des amorces pendant 20 secondes à 63,2 °C, d'une extension pendant 1 minute à 72 °C et d'une extension finale pendant 10 minutes à 72 °C.

7. Procédé selon l'une quelconque des revendications précédentes, dans lequel la seconde PCR multiplex est réalisée avec 0,625 U d'ADN polymérase DFS-Taq, 1 µM de chaque amorce et 5 ng/µL d'échantillon d'ADN, l'activation de l'enzyme est effectuée pendant 2 minutes à 94 °C, suivie de 30 cycles de dénaturation de l'ADN pendant 10 secondes à 94 °C, d'hybridation des amorces pendant 20 secondes à 63 °C, d'une extension pendant 1 minute à 72 °C et d'une extension finale pendant 10 minutes à 72 °C.

8. Procédé selon l'une quelconque des revendications précédentes, dans lequel la troisième PCR multiplex est réalisée avec 1 U d'ADN polymérase, 0,6 µM de chaque amorce, 2 mM de MgCl₂ et 10 ng/µL d'échantillon d'ADN, l'activation de l'enzyme est effectuée pendant 15 minutes à 95 °C, suivie de 30 cycles de dénaturation de l'ADN pendant 30 secondes à 95 °C, d'hybridation des amorces pendant 2 minutes à 65,9 °C, d'une extension pendant 2 minutes à 72 °C et d'une extension finale pendant 10 minutes à 72 °C.

9. Procédé selon l'une quelconque des revendications précédentes, dans lequel les produits des trois PCR multiplex sont combinés dans un rapport de 1:1.

10. Procédé selon l'une quelconque des revendications précédentes, dans lequel la première étape de purification de l'échantillon est effectuée pendant 15 minutes à 37 °C, suivie de 15 minutes à 80 °C pour l'inactivation enzymatique.

11. Procédé selon l'une quelconque des revendications précédentes, dans lequel la réaction d'extension est effectuée avec 25 cycles à 90 °C pendant 10 secondes, à 50 °C pendant 5 secondes, à 60 °C pendant 3 secondes et une étape d'extension finale à 72 °C pendant 2 minutes.

12. Procédé selon l'une quelconque des revendications précédentes, dans lequel la purification du produit de la réaction d'extension est effectuée pendant 1 heure à 37 °C, suivie de 15 minutes à 75 °C pour l'inactivation enzymatique.

13. Procédé selon l'une quelconque des revendications précédentes, dans lequel les produits résultants sont analysés par électrophorèse capillaire.

14. Kit pour le génotypage de polymorphismes mononucléotidiques dans des gènes de l'axe somatotrope qui sont associés à la production et à la qualité du lait ovin, dans lequel le kit comprend le jeu d'amorces défini par les SEQ ID No. 1 à 36, destiné à être utilisé dans le procédé divulgué selon l'une quelconque des revendications 1 à 13.

15. Kit selon la revendication précédentedans lequel les polymorphismes mononucléotidiques sont au moins l'un des polymorphismes choisis dans la liste comprenant : g.597T>C, g.717C>T, g.1024T>C dans le gène GH ; rs1086611503, rs595567866, rs597181420 dans le gène du récepteur de GH ; rs400524445, rs1094731965 dans le gène du récepteur d'IGF1 ; g.23731G>T, rs412263261, rs408430940, rs406266481 dans le gène de la prolactine ; rs604784916, rs600947105 dans le gène du récepteur de PRL ; rs401028781, rs412597723 dans le gène IGF1.
